# EUROPÄISCHE PATENTSCHRIFT

(11) **EP 1 200 050 B1**
(45) Veröffentlichungstag und Bekanntmachung des Hinweises auf die Patenterteilung: **22.09.2004**
(21) Anmeldenummer: 00956364.4
(22) Anmeldetag: 31.07.2000
(51) Int. Cl.: A61K 7/13

(54) **MITTEL ZUM FÄRBEN VON KERATINHALTIGEN FASERN**
DYEING AGENT FOR KERATINOUS FIBRES
AGENT SERVANT A COLORER DES FIBRES KERATINIQUES

(30) Priorität: 06.08.1999 DE 19937289
(43) Veröffentlichungstag der Anmeldung: 02.05.2002
(73) Patentinhaber: Henkel Kommanditgesellschaft auf Aktien, 40589 Düsseldorf-Holthausen (DE)
(72) Erfinder: MÖLLER, Hinrich, 40789 Monheim (DE); OBERKOBUSCH, Doris, 40591 Düsseldorf (DE); HÖFFKES, Horst, 40595 Düsseldorf (DE)
(86) Internationale Anmeldenummer: PCT/EP2000/007405
(87) Internationale Veröffentlichungsnummer: WO 2001/010379

(56) Entgegenhaltungen:
- WO-A-00/38633
- WO-A-01/05359
- WO-A-99/18916
- DE-C- 19 649 242

## Beschreibung

Die Erfindung betrifft ein Mittel zum Färben von keratinhaltigen Fasern, insbesondere menschlichen Haaren, das eine Kombination aus heteroaromatischen Aldehyden und Ketonen und heteroaromatischen Nitroaminen enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasem, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kuppterkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazo-Ionderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2.5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7-und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

In der Patentanmeldung WO-A2-99/18916 werden Haarfärbemittel beschrieben, welche mindestens einen kationischen heterozyklischen Oniumaldehyd und/oder mindestens ein entsprechendes Oniumketon in Kombination mit mindestens einer Verbindung ausgewählt aus Verbindungen mit primärer oder sekundärer Aminogruppe oder Hydroxygruppe, Aminosäuren, Oligopeptiden und CH-aktiven Verbindungen enthalten. Die Verwendung der erfindungsgemäßen heteroaromatischen Nitroamine in Haarfärbemitteln wird in diesem Dokument nicht vorgeschlagen.

Die Verwendung der unten näher beschriebenen eine Kombination aus heteroaromatisehen Aldehyden und heteroaromatischen Nitroaminen zum Färben von keratinhaltigen Fasern ist bislang nicht bekannt.

Aufgabe der vorliegenden Erfindung ist es, Färbemittel für Keratinfasem, insbesondere menschliche Haare, bereitzustellen, die hinsichtlich der Farbtiefe, derGrauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.
Überraschenderweise wurde nun gefunden, daß die Kombination aus den in der Formel I dargestellten heteroaromatischen Aldehyden und Ketonen und heteroaromatischen Nitroaminen der Formel II sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farb-nuancen. Der Einsatz von oxidierenden Agentien soll dabei jedoch nicht prinzipiell ausgeschlossen werden.

Gegenstand der Erfindung ist ein Mittel zum Färben vonkeratinhahigen Fasern, insbesondere menschlichen Haaren, enthaltend eine Kombination aus heteroaromatischen Aldehyden mit der Formel I, 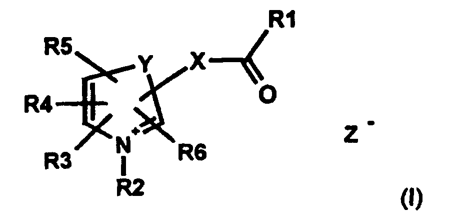 in der
R¹ ein Wasserstoffatom, eine C₁₋₄-Alkyl-, eine Aryl- oder Heteroarylgruppe,
R² eine C₁₋₄-Alkyl-, eine Aryl-, Aralkyl- oder Heteroarylgruppe,
R³, R⁴, R⁵ und R⁶ ein Wasserstoff- oder Halogenatom, eine C₁₋₄-Alkyl-, C₁₋₄-Alkoxyoder C₁₋₄-Hydroxyalkoxygruppe, eine Hydroxy-, Nitro-, Aminogruppe, die durch C₁₋ ₄-Alkylgruppen substituiert sein kann, oder C₁₋₄-Acylgruppe, wobei auch zwei der Reste gemeinsam einen ankondensierten aromatischen Ring oder einer der Reste zusammen mit R¹ oder mit R² einen ankondensierten 5-, 6- oder 7-Ring, der ebenfalls einen ankondensierten aromatischen Ring tragen kann, bilden können,
X eine direkte Bindung, eine gegebenenfalls substituierte Vinylen-, Phenylen- oder Vinylenphenylengruppe,
Y ein Schwefelatom, ein Sauerstoffatom,
die Gruppe CR⁷R⁸, in der R⁷ und R⁸ für eine C₁₋₄-Alkylgruppe stehen,
die Gruppe NR¹⁰, in der R¹⁰ für eine C₁₋₄-Alkylgruppe steht, oder eine gegebenenfalls substituierte Vinylengruppe und
Z Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat oder Tetrachlorzinkat bedeuten,
und heteroaromatischen Nitroaminen mit der Formel II, 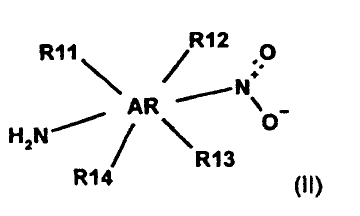 in der
AR Pyridin, Chinolin, Isochinolin, Pyrimidin, Indol, Benzimidazol, Benzothiazol, Indazol, Benzoxazol, Chinoxalin, Chinazolin, Cinnolin und
R¹¹, R¹², R¹³ und R¹⁴ ein Wasserstoffatom, Halogenatom, eine C₁₋₄-Alkyl-, C₁₋₄-Alkoxy-, C₁₋₄-Aminoalkyl-, C₁₋₄-Hydroxyalkoxy-, Hydroxy-, Nitro-, Carboxy-, C₁₋₄-Alkoxycarbonyl-, Sulfo-, Sulfamoyl-, Arylazo- oder Aminogruppe, die durch C₁₋₄-Alkyl- oder C₁₋₄-Hydroxyalkylgruppen substiuiert sein kann, bedeuten,
und/oder ein Reaktionsprodukt aus den Verbindungen mit den Formeln I und 11.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyloder Acetylcellulose und synthetischer Fasern, wie z. B. Polyamid-, Polyacrylnitril-, Polyurethan- und Polyesterfasern verwendet werden.

Bevorzugt eingesetzte Verbindungen mit der Formel I sind die 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium-, 4-Acetyl-1-methylpyridinium-, 2-Acetyl-1-methylpyridinium-, 4-Acetyl-1-methylchinolinium-, 5-Acetyl-1-methylchinolinium-, 6-Acetyl-1-methylchinolinium-, 7-Acetyl-1-methylchinolinium-, 8-Acetyl-1-methylchinolinium-, 9-Formyl-10-methylacridinium-, 4-(2-Formylvinyl)-1-methylpyridinium-, 1,3-Dimethyl-2-(4-formylphenyl)-benzimidazolinium, 1,3-Dimethyl-2-(4-formylphenyl)-imidazolinium-, 2-(4-Formylphenyl)-3-methylbenzothiazolium-, 2-(4-Acetylphenyl)-3-methylbenzothiazolium-, 2-(4-Formylphenyl)-3-methylbenzoxazolium-, 2-(5-Formyl-2-furyl)-3-methylbenzothiazolium-, 2-(5-Formyl-2-furyl)-3-methylbenzothiazolium-, 2-(5-Formyl-2-thienyl)-3-methylbenzothiazolium-, 2-(3-Formylphenyl)-3-methylbenzothiazolium-, 2-(4-Formyl-1-naphthyl)-3-methylbenzothiazolium-, 5-Chlor-2-(4-formylphenyl)-3-methylbenzothiazolium-, 2-(4-Formylphenyl)-3,5-dimethylbenzothiazolium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium-, 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-formylphenyl)-ethenyl]-chinolinium-, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-, 1-Methyl-5-oxo-indeno[1,2-b]pyridinium-, 1-Ethyl-5-oxoindeno[1,2-b]pyridinium-, 1-Benzyl-5-oxo-indeno[1,2-b]pyridinium-, (4-Ethyl-4-azonio-9-fluorenon)-, (4-Benzyl-4-azonio-9-fluorenon)-, 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-salze, insbesondere die Benzosulfonate, p-Toluolfulfonate, Methansulfonate, Perchlorate, Sulfate, Chloride, Bromide, Jodide, Tetrachlorzinkate, Methylsulfate, Trifluormethansulfonate, Hexafluorphosphate, Tetrafluorborate sowie deren beliebigen Gemische.

Als Beispiele für bevorzugt eingesetzte heteroaromatische Nitroamine der Formel II können genannt werden 3-Amino-6-methylamino-2-nitro-pyridin, 6-Nitro-2,5-diaminopyridin und beliebige Gemische der voranstehenden Verbindungen.

Diese Substanzen sind zum großen Teil literaturbekannt oder im Handel erhältlich oder nach bekannten Syntheseverfahren herstellbar.

Die voranstehend genannten Verbindungen mit der Formel I bzw. Formel II werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbemittel, die als färbende Komponente die erfindungsgemäße Kombination allein enthalten, werden bevorzugt für Färbungen im Nuancenbereich von gelb über gelbbraun, orange, braunorange, rot, rotbraun violett bis hin zu blauviolett und schwarz eingesetzt.

Zur Erlangung weiterer und intensiverer Ausfärbungen können die erfindungsgemäßen Mittel können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch vertraglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Kombinationen aus den Verbindungen mit der Formel I und der Formel II gemeinsam zum Einsatz kommen; ebenso können auch mehrere verschiedene Farbverstärker gemeinsam verwendet werden.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, Disperse Violet 1, Disperse Violet 4, Disperse Black 9, Basic Brown 16 und Basic Brown 17 bekannten Verbindungen sowie 2-Amino-6-chloro-4-nitrophenol, 6-Nitro-1,2,3,4-tetrahydrochinoxalin, 4-N-Ethyl-1,4-bis(2-hydroxyethylamino)-2-nitrobenzol-hydrochlorid und 1-Methyt-3-nitro-4-(2-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die erfindungsgemäß enthaltenen Verbindungen mit der Formeln I und II oder die fakultativ enthaltenen Farbverstärker unddirektziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel inwasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3C-Atomen in der Alkanotgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine - COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Triatkylmethylammoniumchioride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 3 und 11, vorzugsweise zwischen 4 und 10.

Ein weiterer Gegenstand der vorliegenden Erfindung betrifft die Verwendung von einer Kombination aus heteroaromatischen Aldehyden und Ketonen mit der Formel I, 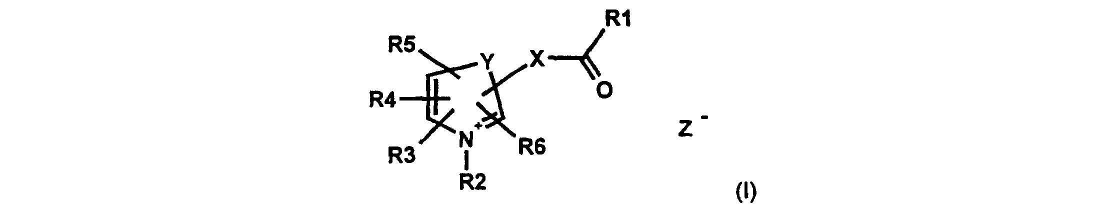 in der
R¹ ein Wasserstoffatom, eine C₁₋₄-Alkyl-, eine Aryl- oder Heteroarylgruppe,
R² eine C₁₋₄-Alkyl-, eine Aryl-, Aralkyl- oder Heteroarylgruppe,
R³, R⁴, R⁵ und R⁶ ein Wasserstoff- oder Halogenatom, eine C₁₋₄-Alkyl-, C₁₋₄-Alkoxyoder C₁₋₄-Hydroxyalkoxygruppe, eine Hydroxy-, Nitro-, Aminogruppe, die durch C₁₋ ₄-Alkylgruppen substituiert sein kann, oder C₁₋₄-Acylgruppe, wobei auch zwei der Reste gemeinsam einen ankondensierten aromatischen Ring oder einer der Reste zusammen mit R¹ oder mit R² einen ankondensierten 5-, 6- oder 7-Ring, der ebenfalls einen ankondensierten aromatischen Ring tragen kann, bilden können,
X eine direkte Bindung, eine gegebenenfalls substituierte Vinylen-, Phenylen- oder Vinylenphenylengruppe,
Y ein Schwefelatom, ein Sauerstoffatom,
die Gruppe CR⁷R⁸, in der R⁷ und R⁸ für eine C₁₋₄-Alkylgruppe stehen,
die Gruppe NR¹⁰, in der R¹⁰ für eine C₁₋₄-Alkylgruppe steht, oder eine gegebenenfalls substituierte Vinylengruppe und
Z⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat oder Tetrachlorzinkat bedeuten,
und heteroaromatischen Nitroaminen mit der Formel II, 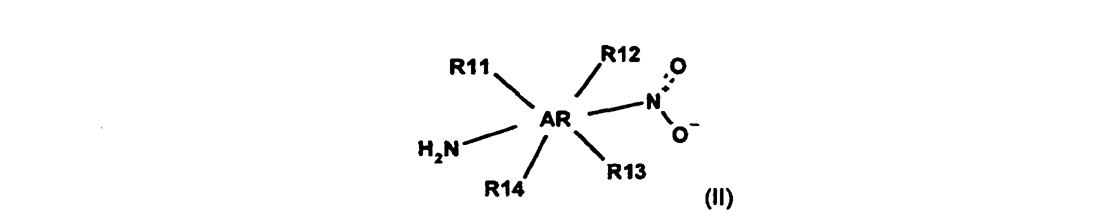 in der
AR Pyridin, Chinolin, Isochinolin, Pyrimidin, Indol, Benzimidazol, Benzothiazol, Indazol, Benzoxazol, Chinoxalin, Chinazolin, Cinnolin und
R¹¹, R¹², R¹³ und R¹⁴ ein Wasserstoffatom, Halogenatom, eine C₁₋₄-Alkyl-, C₁₋₄-Alkoxy-, C₁₋₄-Aminoalkyl-, C₁₋₄-Hydroxyalkoxy-, Hydroxy-, Nitro-, Carboxy-, C₁₋₄-Alkoxycarbonyl-, Sulfo-, Sulfamoyl-, Arylazo- oder Aminogruppe, die durch C₁₋₄-Alkyl- oder C₁₋₄-Hydroxyalkylgruppen substiuiert sein kann, bedeuten,
und/oder eines Reaktionsproduktes aus den Verbindungen mit den Formeln I und II als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend eine Kombination aus heteroaromatischen Aldehyden und Ketonen mit der Formel I, 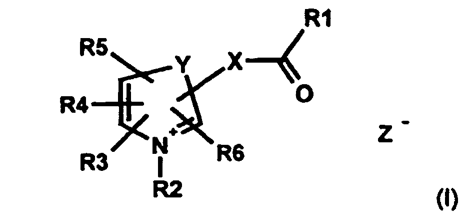 in der
R¹ ein Wasserstoffatom, eine C₁₋₄-Alkyl-, eine Aryl- oder Heteroarylgruppe,
R² eine C₁₋₄-Alkyl-, eine Aryl-, Aralkyl- oder Heteroarylgruppe,
R³, R⁴, R⁵ und R⁶ ein Wasserstoff- oder Halogenatom, eine C₁₋₄-Alkyl-, C₁₋₄-Alkoxyoder C₁₋₄-Hydroxyalkoxygruppe, eine Hydroxy-, Nitro-, Aminogruppe, die durch C₁₋ ₄-Alkylgruppen substituiert sein kann, oder C₁₋₄-Acylgruppe, wobei auch zwei der Reste gemeinsam einen ankondensierten aromatischen Ring oder einer der Reste zusammen mit R¹ oder mit R² einen ankondensierten 5-, 6- oder 7-Ring, der ebenfalls einen ankondensierten aromatischen Ring tragen kann, bilden können,
X eine direkte Bindung, eine gegebenenfalls substituierte Vinylen-, Phenylen- oder Vinylenphenylengruppe,
Y ein Schwefelatom, ein Sauerstoffatom,
die Gruppe CR⁷R⁸, in der R⁷ und R⁸ für eine C₁₋₄-Alkylgruppe stehen,
die Gruppe NR¹⁰, in der R¹⁰ für eine C₁₋₄-Alkylgruppe steht, oder eine gegebenenfalls substituierte Vinylengruppe und
Z⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat oder Tetrachlorzinkat bedeuten,
und heteroaromatischen Nitroaminen mit der Formel II, 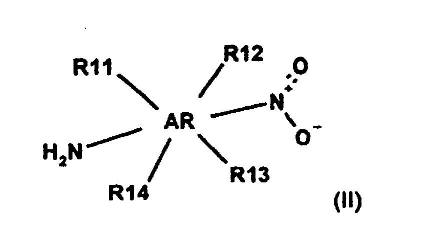 in der
AR Pyridin, Chinolin, Isochinolin, Pyrimidin, Indol, Benzimidazol, Benzothiazol, Indazol, Benzoxazol, Chinoxalin, Chinazolin, Cinnolin, und
R¹¹, R¹², R¹³ und R¹⁴ ein Wasserstoffatom, Halogenatom, eine C₁₋₄-Alkyl-, C₁₋₄-Alkoxy-, C₁₋₄-Aminoalkyl-, C₁₋₄-Hydroxyalkoxy-, Hydroxy-, Nitro-, Carboxy-, C₁₋₄-Alkoxycarbonyl-, Sulfo-, Sulfamoyl-, Arylazo- oder Aminogruppe, die durch C₁₋₄-Alkyl- oder C₁₋₄-Hydroxyalkylgruppen substiuiert sein kann, bedeuten,
und/oder ein Reaktionsprodukt aus diesen Verbindungen
sowie übliche kosmetische Inhaltsstoffe, auf die keratinhaltigen Fasem aufgebracht, einige Zeit, üblicherweise ca. 30 Minuten, auf der Faser belassen und anschließend wieder ausgespült oder mit einem Shampoo ausgewaschen wird.

Die heteroaromatischen Aldehyde und Ketone der Formel I und die heteroaromatischen Nitroamine der Formel II können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die heteroaromatischen Aldehyde und Ketone der Formel I und die heteroaromatischen Nitroamine der Formel II können entweder getrennt oder zusammen gelagert werden, entweder in einer flüssigen bis pastösen Zubereitung (wäßrig oderwasserfrei) oder als trockenes Pulver. Bei der getrennten Lagerung werden die Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5,0 mmol eines aromatischen Aldehyds oder Ketons mit der Formel I, 5,0 mmol eines Nitroamins der Formel II in 25 ml Wasser bei 50°C hergestellt. Die Aufschlämmungen wurden nach Abkühlen auf 30°C miteinander vermischt, mit 5 mmol Natriumacetat und einem Tropfen einer 20-%igen Fettalkylethersulfat-Lösung versetzt und mit verdünnter NaOH oder Salzsäure der pH-Wert ( soweit nicht anders vermerkt 6,0) eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in den nachfolgenden Tabellen wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **Aldehyd** | **Nitroamin** | Farbe | Intensität |
|---|---|---|---|
| 4-Benzoyl-1-methyl-pyridinium-methansulfonat | 2-Nitro-3-amino-6-aminomethylpyridin (Azarot) | orange | ++ |
| 4-Formyl-1-methyl-pyridinium-benzolsulfonat | 2-Nitro-3-amino-6-aminomethylpyridin (Azarot) | braunrosa | +(+) |
| 4-Formyl-1-methyl-chinolinium-p-toluolsulfonat | 2-Amino-6-nitrobenzothiazol* | orangebraun | ++ |
| 4-Formyl-1-methyl-pyridinium-benzolsulfonat | 2-Amino-6-nitrobenzothiazol* | hellbraun | + |

| | | | |
|---|---|---|---|
| * mit äquimolarer Menge an Piperidin als Farbverstärker bei pH=9,0 | | | |

## Patentansprüche

1. Mittel zum Färben von keratinhattigen Fasern, insbesondere menschlichen Haaren, enthaltend als färbende Komponente eine Kombination aus heteroaromatischen Aldehyden und Ketonen mit der Formel I, 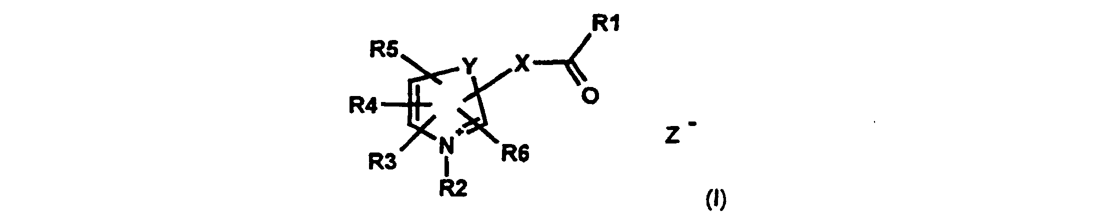 in der
R¹ ein Wasserstoffatom, eine C₁₋₄-Alkyl-, eine Aryl- oder Heteroarylgruppe,
R² eine C₁₋₄-Alkyl-, eine Aryl-, Aralkyl- oder Heteroarylgruppe,
R³, R⁴, R⁵ und R⁶ ein Wasserstoff- oder Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, oder C₁₋₄-Acylgruppe, wobei auch zwei der Reste gemeinsam einen ankondensierten aromatischen Ring oder einer der Reste zusammen mit R¹ oder mit R² einen ankondensierten 5-, 6- oder 7-Ring, der ebenfalls einen ankondensierten aromatischen Ring tragen kann, bilden können,
X eine direkte Bindung, eine gegebenenfalls substituierte Vinylen-, Phenylenoder Vinylenphenylengruppe,
Y ein Schwefelatom, ein Sauerstoffatom,
die Gruppe CR⁷R⁸, in der R⁷ und R⁸ für eine C₁₋₄-Alkylgruppe stehen,
die Gruppe NR¹⁰, in der R¹⁰ für eine C₁₋₄-Alkylgruppe steht, oder eine gegebenenfalls substituierte Vinylengruppe und
Z⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat oder Tetrachlorzinkat bedeuten,
und heteroaromatischen Nitroaminen mit der Formel II, 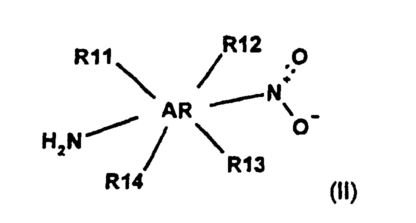 in der
AR Pyridin, Chinolin, Isochinolin, Pyrimidin, Indol, Benzimidazol, Benzothiazol, Indazol, Benzoxazol, Chinoxalin, Chinazolin, Cinnolin, und
R¹¹, R¹², R¹³ und R¹⁴ ein Wasserstoffatom, Halogenatom, eine C₁₋₄-Alkyl-, C₁₋₄-Alkoxy-, C₁₋₄-Aminoalkyl-, C₁₋₄-Hydroxyalkoxy-, Hydroxy-, Nitro-, Carboxy-, C₁₋ ₄-Alkoxycarbonyl-, Sulfo-, Sulfamoyl-, Arylazo- oder Aminogruppe, die durch C₁₋₄-Alkyl- oder C₁₋₄-Hydroxyalkylgruppen substiuiert sein kann, bedeuten
und/oder ein Reaktionsprodukt aus den Verbindungen mit der Formel I und II

2. Mittel nach Anspruch 1, **dadurch gekennzeichnet, daß** als Verbindungen mit der Formel I 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium-, 4-Acetyl-1-methylpyridinium-, 2-Acetyl-1-methylpyridinium-, 4-Acetyl-1-methylchinolinium-, 5-Acetyl-1-methylchinolinium-, 6-Acetyl-1-methylchinolinium-, 7-Acetyl-1-methylchinolinium-, 8-Acetyl-1-methylchinolinium-, 9-Formyl-10-methylacridinium-, 4-(2-Formylvinyl)-1-methylpyridinium-, 1,3-Dimethyl-2-(4-formylphenyl)-benzimidazolinium, 1,3-Dimethyl-2-(4-formylphenyl)-imidazolinium-, 2-(4-Formylphenyl)-3-methylbenzothiazolium-, 2-(4-Acetylphenyl)-3-methylbenzothiazolium-, 2-(4-Formylphenyl)-3-methylbenzoxazolium-, 2-(5-Formyl-2-furyl)-3-methylbenzothiazolium-, 2-(5-Formyl-2-furyl)-3-methylbenzothiazolium-, 2-(5-Formyl-2-thienyl)-3-methylbenzothiazolium-, 2-(3-Formylphenyl)-3-methylbenzothiazolium-, 2-(4-Formyl-1-naphthyl)-3-methylbenzothiazolium-, 5-Chlor-2-(4-formylphenyl)-3-methylbenzothiazolium-, 2-(4-Formylphenyl)-3,5-dimethylbenzothiazolium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium-, 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-formylphenyl)-ethenyl]-chinolinium-, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzathiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-, 1-Methyl-5-oxo-indeno[1,2-b]pyridinium-, 1-Ethyl-5-oxo-indeno[1,2-b]pyridinium-, 1-Benzyl-5-oxo-indeno[1,2-b]pyridinium-, (4-Ethyl-4-azonio-9-fluorenon)-, (4-Benzyl-4-azonio-9-fluorenon)-, 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-salze, insbesondere die Benzosulfonate, p-Toluolfulfonate, Methansulfonate, Perchlorate, Sulfate, Chloride, Bromide, Iodide, Tetrachlorzinkate, Methylsulfate, Trifluormethansulfonate, Hexafluorphosphate, Tetrafluorborate sowie deren beliebigen Gemische eingesetzt werden.

3. Mittel nach Anspruch 1 oder 2, **dadurch gekennzeichnet, daß** als Verbindung mit der Formel II 3-Amino-6-methylamino-2-nitro-pyridin, 6-Nitro-2,5-diaminopyridin und beliebige Gemische der voranstehenden Verbindungen.

4. Mittel nach einem der Ansprüche 1 bis 3, **dadurch gekennzeichnet, daß** die heteroaromatischen Aldehyde und Ketone und heteroaromatischen Nitroamine mit der Formel II jeweils in einer Menge von 0,03 bis 65 mmol, insbesondere von 1 bis 40 mmol, bezogen auf 100 g des gesamten Färbemittels, enthalten sind.

5. Mittel nach einem der Ansprüche 1 bis 4, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

6. Mittel nach einem der Ansprüche 1 bis 5, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

7. Mittel nach einem der Ansprüche 1 bis 6, **dadurch gekennzeichnet, daß** Ammoniumoder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

8. Mittel nach einem der Ansprüche 1 bis 7, **dadurch gekennzeichnet, daß** es Oxidationsmittel, insbesondere H₂O₂, in einer Menge von 0,01 bis 6 Gew.-%, bezogen auf die Anwendungslösung, enthält.

9. Mittel nach einem der Ansprüche 1 bis 8, **dadurch gekennzeichnet, daß** es anionische, zwitterionische oder nichtionische Tenside enthält.

10. Verwendung von einer Kombination aus heteroaromatischen Aldehyden und Ketonen mit der Formel I, 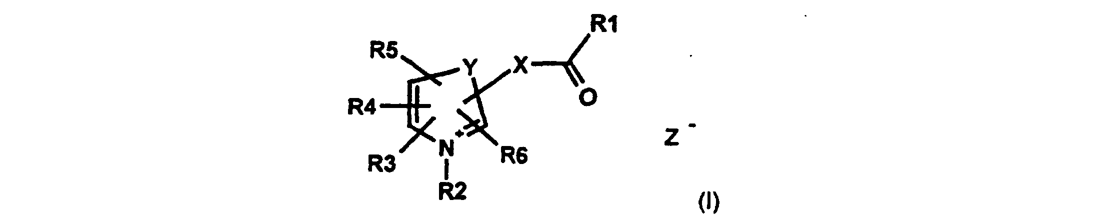 in der
R¹ ein Wasserstoffatom, eine C₁₋₄-Alkyl-, eine Aryl- oder Heteroarylgruppe,
R² eine C₁₋₄-Alkyl-, eine Aryl-, Aralkyl- oder Heteroarylgruppe,
R³, R⁴, R⁵ und R⁶ ein Wasserstoff- oder Halogenatom, eine C₁₋₄-Alkyl-, C₁₋₄-Alkoxy- oder C₁₋₄-Hydroxyalkoxygruppe, eine Hydroxy-, Nitro-, Aminogruppe,
die durch C₁₋₄-Alkylgruppen substituiert sein kann, oder C₁₋₄-Acylgruppe, wobei auch zwei der Reste gemeinsam einen ankondensierten aromatischen Ring oder einer der Reste zusammen mit R¹ oder mit R² einen ankondensierten 5-, 6- oder 7-Ring, der ebenfalls einen ankondensierten aromatischen Ring tragen kann, bilden können,
X eine direkte Bindung, eine gegebenenfalls substituierte Vinylen-, Phenylenoder Vinylenphenylengruppe,
Y ein Schwefelatom, ein Sauerstoffatom,
die Gruppe CR⁷R⁸, in der R⁷ und R⁸ für eine C₁₋₄-Alkylgruppe stehen,
die Gruppe NR¹⁰, in der R¹⁰ für eine C₁₋₄-Alkylgruppe steht, oder eine gegebenenfalls substituierte Vinylengruppe und
Z⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat oder Tetrachlorzinkat bedeuten,
und heteroaromatischen Nitroaminen mit der Formel II, 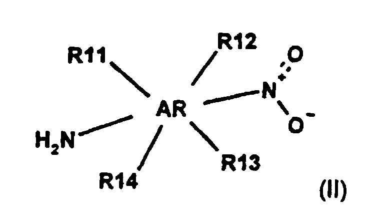 in der
AR Pyridin, Chinolin, Isochinolin, Pyrimidin, Indol, Benzimidazol, Benzothiazol, Indazol, Benzoxazol, Chinoxalin, Chinazolin, Cinnolin, und
R¹¹, R¹², R¹³ und R¹⁴ ein Wasserstoffatom, Halogenatom, eine C₁₋₄-Alkyl-, C₁₋₄-Alkoxy-, C₁₋₄-Aminoalkyl-, C₁₋₄-Hydroxyalkoxy-, Hydroxy-, Nitro-, Carboxy-, C₁₋ ₄-Alkoxycarbonyl-, Sutfo-, Sulfamoyl-, Arylazo- oder Aminogruppe, die durch C₁₋₄-Alkyl- oder C₁₋₄-Hydroxyalkylgruppen substiuiert sein kann, bedeuten,
und/oder eines Reaktionsproduktes aus den Verbindungen mit der Formel I und II als eine färbende Komponente in Oxidationshaarfärbemitteln.

11. Verfahren zum Färben von keratinhaltigen Fasern, insbesondere menschlichen Haaren, worin ein Färbemittel, enthaltend mindestens eine Kombination aus heteroaromatischen Aldehyden und Ketonen der Formel I, 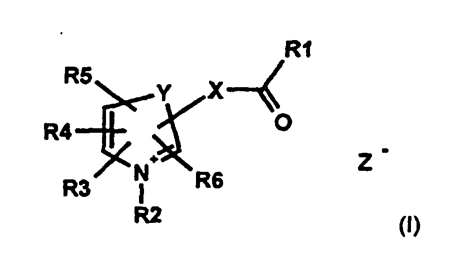 in der
R¹ ein Wasserstoffatom, eine C₁₋₄-Alkyl-, eine Aryl- oder Heteroarylgruppe,
R² eine C₁₋₄-Alkyl-, eine Aryl-, Aralkyl- oder Heteroarylgruppe,
R³, R⁴, R⁵ und R⁶ ein Wasserstoff- oder Halogenatom, eine C₁₋₄-Alkyl-, C₁₋₄-Alkoxy- oder C₁₋₄-Hydroxyalkoxygruppe, eine Hydroxy-, Nitro-, Aminogruppe,
die durch C₁₋₄-Alkylgruppen substituiert sein kann, oder C₁₋₄-Acylgruppe, wobei auch zwei der Reste gemeinsam einen ankondensierten aromatischen Ring oder einer der Reste zusammen mit R¹ oder mit R² einen ankondensierten 5-, 6- oder 7-Ring, der ebenfalls einen ankondensierten aromatischen Ring tragen kann, bilden können,
X eine direkte Bindung, eine gegebenenfalls substituierte Vinylen-, Phenylenoder Vinylenphenylengruppe,
Y ein Schwefelatom, ein Sauerstoffatom,
die Gruppe CR⁷R⁸, in der R⁷ und R⁸ für eine C₁₋₄-Alkylgruppe stehen,
die Gruppe NR¹⁰, in der R¹⁰ für eine C₁₋₄-Alkylgruppe steht, oder eine gegebenenfalls substituierte Vinylengruppe und
Z⁻ Halogenid, C₁₋₄-Alkylsulfat, Arensulfat, C₁₋₄-Alkansulfonat, C₁₋₄-Perfluoralkansulfonat, Perhalogenat, Sulfat, Hydrogensulfat, Tetrafluorborat, Hexafluorphosphat oder Tetrachlorzinkat bedeuten,
und heteroaromatischen Nitroaminen mit der Formel II, 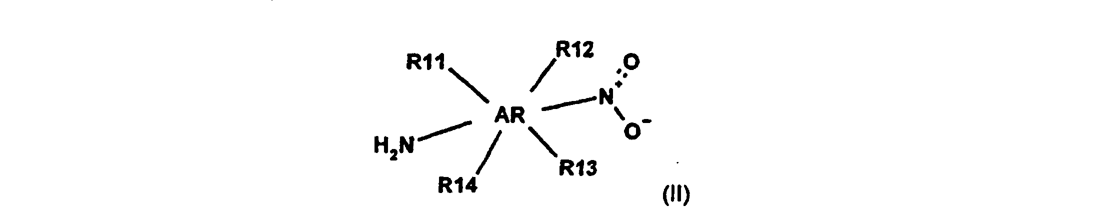 in der
AR Pyridin, Chinolin, Isochinolin, Pyrimidin, Indol, Benzimidazol, Benzothiazol, Indazol, Benzoxazol, Chinoxalin, Chinazolin, Cinnolin, und
R¹¹, R¹², R¹³ und R¹⁴ ein Wasserstoffatom, Halogenatom, eine C₁₋₄-Alkyl-, C₁₋₄-Alkoxy-, C₁₋₄-Aminoalkyl-, C₁₋₄-Hydroxyalkoxy-, Hydroxy-, Nitro-, Carboxy-, C₁₋ ₄-Alkoxycarbonyl-, Sulfo-, Sulfamoyl-, Arylazo- oder Aminogruppe, die durch C₁₋₄-Alkyl- oder C₁₋₄-Hydroxyalkylgruppen substiuiert sein kann bedeuten,
und/oder ein Reaktionsprodukt aus den diesen Verbindungen sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. A composition for colouring keratin-containing fibres, more particularly human hair, containing as a colouring component a combination of heteroaromatic aldehydes and ketones corresponding to formula I: 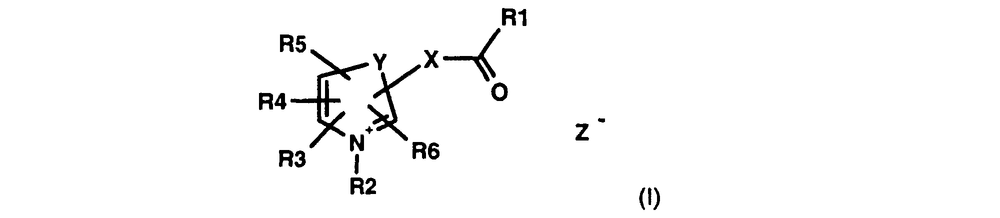 in which
R¹ is a hydrogen atom, a C₁₋₄ alkyl group, an aryl or heteroaryl group,
R² is a C₁₋₄ alkyl group, an aryl, aralkyl or heteroaryl group,
R³, R⁴, R⁵ and R⁶ represent a hydrogen or halogen atom, a C₁₋₄ alkyl, C₁₋₄ alkoxy or C₁₋₄ hydroxyalkoxy group, a hydroxy, nitro or amino group, which may be substituted by C₁₋₄ alkyl groups, or a C₁₋₄ acyl group; two of the substituents together may also form a fused aromatic ring or one of the substituents together with with R¹ or with R² may form a fused 5-, 6- or 7-membered ring which may also carry a fused aromatic ring,
X is a direct bond, an optionally substituted vinylene, phenylene or vinylenephenylene group,
Y is a sulfur atom, an oxygen atom, the group CR⁷R⁸, where R⁷ and R⁸ represent a C₁₋₄ alkyl group, the group NR¹⁰, where R¹⁰ is a C₁₋₄ alkyl group, or an optionally substituted vinylene group and
Z⁻ is halide, C₁₋₄ alkyl sulfate, arene sulfate, C₁₋₄ alkanesulfonate, C₁₋₄ perfluoroalkane sulfonate, perhalogenate, sulfate, hydrogen sulfate, tetrafluoroborate, hexafluorophosphate or tetrachlorozincate,
and heteroaromatic nitroamines corresponding to formula II: 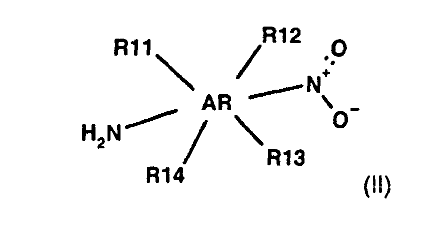 in which
AR represents pyridine, quinoline, isoquinoline, pyrimidine, indole, benzimidazole, benzothiazole, indazole, benzoxazole, quinoxaline, quinazoline, cinnoline and
R¹¹, R¹², R¹³ and R¹⁴ represent a hydrogen atom, a halogen atom, a C₁₋₄ alkyl, C₁₋₄ alkoxy, C₁₋₄ aminoalkyl, C₁₋₄ hydroxyalkoxy, hydroxy, nitro, carboxy, C₁₋₄ alkoxycarbonyl, sulfo, sulfamoyl, arylazo or amino group which may be substituted by C₁₋₄ alkyl or C₁₋₄ hydroxyalkyl groups,
and/or a reaction product of the compounds corresponding to formulae I and II.

2. A composition as claimed in claim 1, **characterized in that** 4-formyl-1-methylpyridinium, 2-formyl-1-methylpyridinium, 4-formyl-1-ethylpyridinium, 2-formyl-1-ethylpyridinium, 4-formyl-1-benzylpyridinium, 2-formyl-1-benzylpyridinium, 4-formyl-1,2-dimethylpyridinium, 4-formyl-1,3-dimethylpyridinium, 4-formyl-1-methylquinolinium, 2-formyl-1-methylquinolinium, 5-formyl-1-methylquinolinium, 6-formyl-1-methylquinolinium, 7-formyl-1-methylquinolinium, 8-formyl-1-methylquinolinium, 4-acetyl-1-methylpyridinium, 2-acetyl-1-methylpyridinium, 4-acetyl-1-methylquinolinium, 5-acetyl-1-methyl-quinolinium, 6-acetyl-1-methylquinolinium, 7-acetyl-1-methylquinolinium, 8-acetyl-1-methylquinolinium, 9-formyl-10-methylacridinium, 4-(2-formylvinyl)-1-methylpyridinium, 1,3-dimethyl-2-(4-formylphenyl)-benzimidazolinium, 1,3-dimethyl-2-(4-formylphenyl)-imidazolinium, 2-(4-formylphenyl)-3-methylbenzothiazolium, 2-(4-acetylphenyl)-3-methylbenzothiazolium, 2-(4-formylphenyl)-3-methylbenzoxazolium, 2-(5-formyl-2-furyl)-3-methylbenzothiazolium, 2-(5-formyl-2-furyl)-3-methylbenzothiazolium, 2-(5-formyl-2-thienyl)-3-methylbenzothiazolium, 2-(3-formylphenyl)-3-methyl-benzothiazolium, 2-(4-formyl-1-naphthyl)-3-methylbenzothiazolium, 5-chloro-2-(4-formylphenyl)-3-methylbenzothiazolium, 2-(4-formylphenyl)-3,5-dimethylbenzothiazolium, 1-methyl-2-[2-(4-formylphenyl)-ethenyl]-pyridinium, 1-methyl-4-[2-(4-acetylphenyl)-ethenyl]-pyridinium-, 1-benzyl-4-[2-(4-formylphenyl)-ethenyl]-pyridinium-, 1-methyl-4-[2-(4-formylphenyl)-ethenyl]-pyridinium-, 1-methyl-2-[2-(4-formylphenyl)-ethenyl]-pyridinium-, 1-methyl-4-[2-(4-formylphenyl)-ethenyl]-quinolinium-, 1-methyl-2-[2-(4-formyl-phenyl)-ethenyl]-quinolinium-, 1-methyl-2-[2-(5-formyl-2-furyl)-ethenyl]-quinolinium-, 1-methyl-2-[2-(5-formyl-2-thienyl)-ethenyl]-quinolinium, 1-methyl-2-[2-(4-formylphenyl)-ethenyl]-benzothiazolinium, 1,3-dimethyl-2-[2-(4-formylphenyl)-ethenyl]-benzimidazolinium, 1,3-dimethyl-2-[2-(4-formyl-phenyl)-ethenyl]-imidazolinium, 1-methyl-5-oxo-indeno[1,2-b]pyridinium, 1-ethyl-5-oxo-indeno[1,2-b]pyridinium, 1-benzyl-5-oxo-indeno[1,2-b]pyridinium, (4-ethyl-4-azonio-9-fluorenone), (4-benzyl-4-azonio-9-fluorenone), 2-methyl-5-oxo-indeno[1,2-c]pyridinium, 2-methyl-9-oxo-indeno[2,1-c]pyridinium, 1-methyl-9-oxo-indeno[2,1-b]pyridinium salts, more particularly the benzenesulfonates, p-toluenesulfonates, methanesulfonates, perchlorates, sulfates, chlorides, bromides, iodides, tetrachlorozincates, methyl sulfates, trifluoromethanesulfonates, hexafluorophosphates, tetrafluroborates and mixtures thereof are used as the compounds corresponding to formula I.

3. A composition as claimed in claim 1 or 2, **characterized in that** 3-amino-6-methylamino-2-nitropyridine, 6-nitro-2,5-diaminopyridine and mixtures of the above are used as the compound corresponding to formula II.

4. A composition as claimed in any of claims 1 to 3, **characterized in that** the heteroaromatic aldehydes and ketones and the heteroaromatic nitroamines are used in quantities of 0.03 to 65 mmol and more particularly 1 to 40 mmol, based on 100 g of the colorant as a whole.

5. A composition as claimed in any of claims 1 to 4, **characterized in that** it contains colour intensifiers selected from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methyl imidazole, histidine, pyrrolidine, proline, pyrrolidone, pyrrolidone-5-carboxylic acid, pyrazole, 1,2,4-triazole, piperazidine or mixtures thereof.

6. A composition as claimed in any of claims 1 to 5, **characterized in that** it contains substantive dyes from the group of nitrophenylenediamines, nitroaminopenols, anthraquinones or indophenols, preferably in a quantity of 0.01 to 20% by weight, based on the colorant as a whole.

7. A composition as claimed in any of claims 1 to 6, **characterized in that** ammonium or metal salts selected from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc are added.

8. A composition as claimed in any of claims 1 to 7, **characterized in that** it contains oxidizing agents, more particularly H₂O₂, in a quantity of 0.01 to 6% by weight, based on the solution applied.

9. A composition as claimed in any of claims 1 to 8, **characterized in that** it contains anionic, zwitterionic or nonionic surfactants.

10. The use of a combination of heteroaromatic aldehydes and ketones corresponding to formula I: 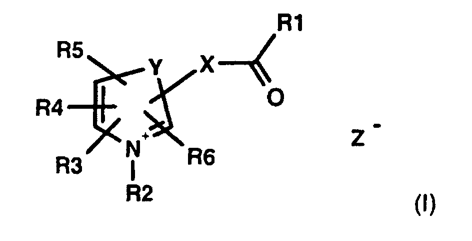 in which
R¹ is a hydrogen atom, a C₁₋₄ alkyl group, an aryl or heteroaryl group,
R² is a C₁₋₄ alkyl group, an aryl, aralkyl or heteroaryl group,
R³, R⁴, R⁵ and R⁶ represent a hydrogen or halogen atom, a C₁₋₄ alkyl, C₁₋₄ alkoxy or C₁₋₄ hydroxyalkoxy group, a hydroxy, nitro or amino group, which may be substituted by C₁₋₄ alkyl groups, or a C₁₋₄ acyl group; two of the substituents together may also form a fused aromatic ring or one of the substituents together with with R¹ or with R² may form a fused 5-, 6- or 7-membered ring which may also carry a fused aromatic ring,
X is a direct bond, an optionally substituted vinylene, phenylene or vinylenephenylene group,
Y is a sulfur atom, an oxygen atom, the group CR⁷R⁸, where R⁷ and R⁸ represent a C₁₋₄ alkyl group, the group NR¹⁰, where R¹⁰ is a C₁₋₄ alkyl group, or an optionally substituted vinylene group and
Z⁻ is halide, C₁₋₄ alkyl sulfate, arene sulfate, C₁₋₄ alkanesulfonate, C₁₋₄ perfluoroalkane sulfonate, perhalogenate, sulfate, hydrogen sulfate, tetrafluoroborate, hexafluorophosphate or tetrachlorozincate,
and heteroaromatic nitroamines corresponding to formula II: 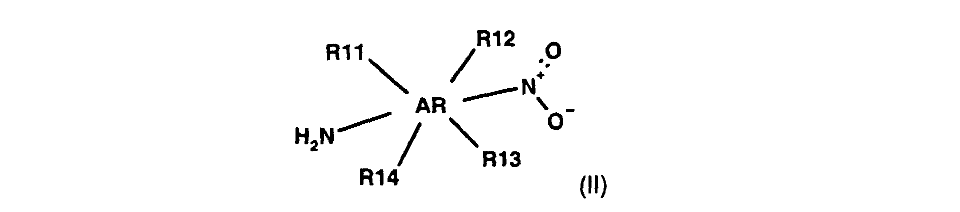 in which
AR represents pyridine, quinoline, isoquinoline, pyrimidine, indole, benzimidazole, benzothiazole, indazole, benzoxazole, quinoxaline, quinazoline, cinnoline and
R¹¹, R¹², R¹³ and R¹⁴ represent a hydrogen atom, a halogen atom, a C₁₋₄ alkyl, C₁₋₄ alkoxy, C₁₋₄ aminoalkyl, C₁₋₄ hydroxyalkoxy, hydroxy, nitro, carboxy, C₁₋₄ alkoxycarbonyl, sulfo, sulfamoyl, arylazo or amino group which may be substituted by C₁₋₄ alkyl or C₁₋₄ hydroxyalkyl groups,
and/or a reaction product of the compounds corresponding to formulae I and II,
as a colouring component in oxidation hair colorants.

11. A process for colouring keratin-containing fibres, more partiularly human hair, in which a colorant containing at least one combination of heteroaromatic aldehydes and ketones corresponding to formula I: 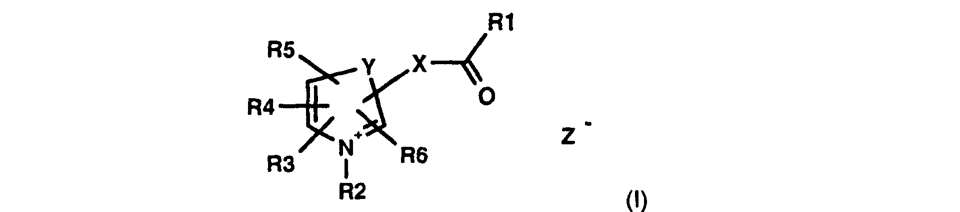 in which
R¹ is a hydrogen atom, a C₁₋₄ alkyl group, an aryl or heteroaryl group,
R² is a C₁₋₄ alkyl group, an aryl, aralkyl or heteroaryl group,
R³, R⁴, R⁵ and R⁶ represent a hydrogen or halogen atom, a C₁₋₄ alkyl, C₁₋₄ alkoxy or C₁₋₄ hydroxyalkoxy group, a hydroxy, nitro or amino group, which may be substituted by C₁₋₄ alkyl groups, or a C₁₋₄ acyl group; two of the substituents together may also form a fused aromatic ring or one of the substituents together with with R' or with R² may form a fused 5-, 6- or 7-membered ring which may also carry a fused aromatic ring,
X is a direct bond, an optionally substituted vinylene, phenylene or vinylenephenylene group,
Y is a sulfur atom, an oxygen atom, the group CR⁷R⁸, where R⁷ and R⁸ represent a C₁₋₄ alkyl group, the group NR¹⁰, where R¹⁰ is a C₁₋₄ alkyl group, or an optionally substituted vinylene group and
Z- is halide, C₁₋₄ alkyl sulfate, arene sulfate, C₁₋₄ alkanesulfonate, C₁₋₄ perfluoroalkane sulfonate, perhalogenate, sulfate, hydrogen sulfate, tetrafluoroborate, hexafluorophosphate or tetrachlorozincate,
and heteroaromatic nitroamines corresponding to formula II: 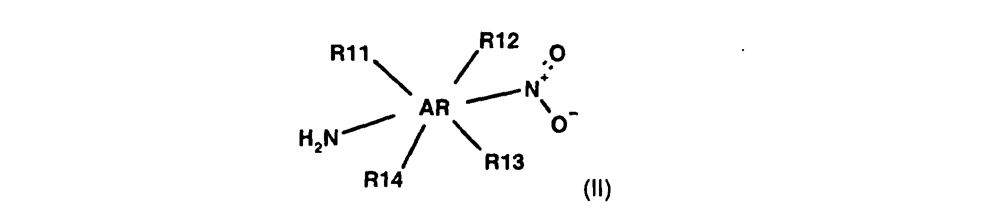 in which
AR represents pyridine, quinoline, isoquinoline, pyrimidine, indole, benzimidazole, benzothiazole, indazole, benzoxazole, quinoxaline, quinazoline, cinnoline and
R¹¹, R¹², R¹³ and R¹⁴ represent a hydrogen atom, a halogen atom, a C₁₋₄ alkyl, C₁₋₄ alkoxy, C₁₋₄ aminoalkyl, C₁₋₄ hydroxyalkoxy, hydroxy, nitro, carboxy, C₁₋₄ alkoxycarbonyl, sulfo, sulfamoyl, arylazo or amino group which may be substituted by C₁₋₄ alkyl or C₁₋₄ hydroxyalkyl groups,
and/or a reaction product of these compounds and typical cosmetic ingredients is applied to the keratin-containing fibres, left thereon for a while, usually about 30 minutes, and then rinsed out or washed out with a shampoo.

## Revendications

1. Agent pour colorer les fibres kératiniques, en particulier les cheveux humains, contenant en tant que composant colorant, une combinaison d'aldéhydes et de cétones hétéroaromatiques de formule I, 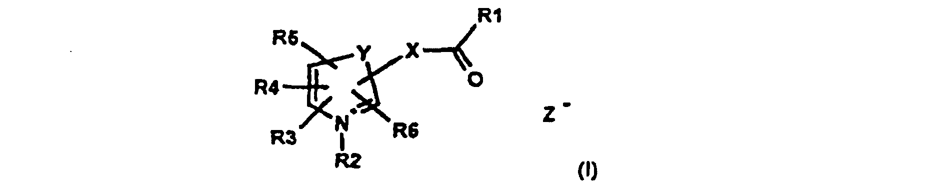 dans laquelle
R¹ représente un atome d'hydrogène, un groupe alkyle en C₁ à C₄, un groupe aryle, ou hétéroaryle,
R² représente un groupe alkyle en C₁ à C₄, un groupe aryle, aralkyle ou hétéroaryle,
R³, R⁴, R⁵ et R⁶ représentent un atome d'hydrogène ou d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyles en C₁ à C₄, ou un groupe acyle en C₁ à C₄, deux des résidus pouvant aussi former conjointement un noyau aromatique condensé ou bien un des résidus conjointement avec R¹ou avec R² pouvant former un cycle condensé à 5, 6, ou 7 maillons, qui peut porter également un noyau aromatique condensé,
X représente une liaison directe, un groupe vinylène, phénylène, ou vinylènephénylène, éventuellement substitué,
Y représente un atome de soufre, un atome d'oxygène,
le groupe CR⁷R⁸, dans lequel R⁷ et R⁸ représentent un groupe alkyle en C₁ à C₄, le groupe NR¹⁰, dans lequel R¹⁰ représente un groupe alkyle en C₁ à C₄, ou un groupe vinylène éventuellement substitué, et
Z⁻ représente un ion halogénure, alkylsulfate en C₁ à C₄, sulfate d'arène, alcane-sulfonate en C₁ à C₄, perfluoroalcane-sulfonate en C₁ à C₄, perhalogénate, sulfate, hydrogénosulfate, tétrafluoroborate, hexafluorophosphate, ou tétrachlorozincate,
et de nitroamines hétéroaromatiques de formule II, 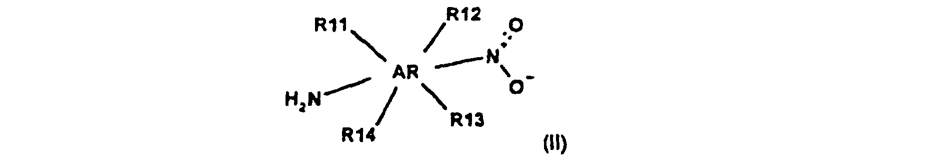 dans laquelle
AR représente un groupe pyridine, quinoléine, isoquinoléine, pyrimidine, indole, benzimidazole, benzothiazole, indazole, benzoxazole, quinoxaline, quinazoline, cinnoline, et
R¹¹, R¹², R¹³, et R¹⁴ représentent un atome d'hydrogène, un atome d'halogène, un groupe alkyle en C₁ à C₄, alcoxy en C₁ à C₄, aminoalkyle en C₁ à C₄, hydroxyalcoxy en C₁ à C₄, hydroxy, nitro, carboxy, (alcoxy en C₁ à C₄)-carbonyle, sulfo, sulfamoyle, arylazo ou amino, qui peut être substitué par des groupes alkyle en C₁ à C₄ ou hydroxyalkyle en C₁ à C₄,
et/ou un produit de réaction issu des composés de formule I et II.

2. Agent selon la revendication 1, **caractérisé en ce que**, on met en oeuvre en tant que composés de formule I, les sels de 4-formyl-1-méthylpyridinium, 2-formyl-1-méthylpyridinium, 4-formyl-1-éthylpyridinium, 2-formyl-1-éthyl-pyridinium, 4-formyl-1-benzylpyridinium, 2-formyl-1-benzyl-pyridinium, 4-formyl-1,2-diméthylpyridinium, 4-formyl-1,3-diméthylpyridinium, 4-formyl-1-méthyl-quinoléinium, 2-formyl-1-méthylquinoléinium, 5-formyl-1-méthylquinoléinium, 6-formyl-1-méthylquinoléinium, 7-formyl-1-méthyl-quinoléinium, 8-formyl-1-méthylquinoléinium, 4-acétyl-1-méthylpyridinium, 2-acétyl-1-méthylpyridinium, 4-acétyl-1-méthylquinoléinium, 5-acétyl-1-méthyl-quinoléinium, 6-acétyl-1-méthylquinoléinium, 7-acétyl-1-méthylquinoléinium, 8-acétyl-1-méthyl-quinoléinium, 9-formyl-10-méthylacridinium, 4-(2-formylvinyl)-1-méthyl-pyridinium, 1,3-diméthyl-2-(4-formylphényl)-benzimidazolinium, 1,3-diméthyl-2-(4-formylphényl)-imidazolinium, 2-(4-formylphényl)-3-méthyl-benzothiazolium, 2-(4-acétylphényl)-3-méthylbenzothiazolium, 2-(4-formyl-phényl)-3-méthyl-benzoxazolium, 2-(5-formyl-2-furyl)-3-méthylbenzothiazolium, 2-(5-formyl-2-furyl)-3-méthylbenzothiazolium, 2-(5-formyl-2-thiényl)-3-méthyl-benzothiazolium, 2-(3-formylphényl)-3-méthylbenzothiazolium, 2-(4-formyl-1-naphtyl)-3-méthyl-benzothiazolium, 5-chloro-2-(4-formylphényl)-3-méthyl-benzothiazolium, 2-(4-formylphényl)-3,5-diméthylbenzothiazolium, 1-méthy)-2-[2-(4-formyl-phényl)-éthényl]-pyridinium, 1-méthyl-4-[2-(4-acétylphényl)-éthényl]-pyridinium, 1-benzyl-4-[2-(4-formylphényl)-éthényl]-pyridinium, 1-méthyl-4-[2-(4-formyl-phényl)-éthényl]-pyridinium, 1-méthyl-2-[2-(4-formyl-phényl)-éthényl]-pyridinium, 1-méthyl-4-[2-(4-formylphényl-éthényl]-quinoléinium, 1-méthyl-2-[2-(4-formyl-phényl)-éthényl]-quinoléinium, 1-méthyl-2-[2-(5-formyl-2-furyl)-éthényl]-quinoléinium, 1-méthyl-2-[2-(5-formyl-2-thiényl)-éthényl]-quinoléinium, 1-méthyl-2-[2-(4-formylphényl)-éthényl]-benzo-thiazolinium, 1,3-diméthyl-2-[2-(4-formyl-phényl)-éthényl]-benzimidazolinium, 1,3-diméthyl-2-[2-(4-formylphényl)-éthényl]-imidazolinium, 1-méthyl-5-oxo-indéno[1,2-b]pyridinium, 1-éthyl-5-oxo-indéno[1,2-b]pyridinium, 1-benzyl-5-oxo-indéno-[1,2-b]pyridinium, (4-éthyl-4-azonio-9-fluorénone), (4-benzyl-4-azonio-9-fluorénone), 2-méthyl-5-oxo-indéno-[1,2-c]pyridinium, 2-méthyl-9-oxo-indéno-[2,1c]pyridinium, 1-méthyl-9-oxo-indéno-[2,1-b]pyridinium, en particulier les benzosulfonates, les p-toluènesulfonates, les méthane-sulfonates, les perchlorates, les sulfates, les chlorures, les bromures, les iodures, les tétrachlorozincates, les méthylsulfates, les trifluorométhane-sulfonates, les hexafluorophosphates, les tétrafluoro-borates, ainsi que leurs mélanges quelconques.

3. Agent selon la revendication 1 ou 2, **caractérisé en ce que** la 3-amino-6-méthylamino-2-nitro-pyridine, la 6-nitro-2,5-diaminopyridine, et des mélanges quelconques des composés précédents y sont contenus, en tant que composé de formule II.

4. Agent selon l'une quelconque des revendications 1 à 3, **caractérisé en ce que** les aldéhydes et cétones hétéroaromatiques et les nitroamines hétéroaromatiques de formule II y sont contenues respectivement en une quantité de 0,03 à 65 mmoles, en particulier de 1 à 40 mmoles, par rapport aux 100 g du colorant total.

5. Agent selon l'une quelconque des revendications 1 à 4, **caractérisé en ce qu'**il contient des substances auxochromes, choisies dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

6. Agent selon l'une quelconque des revendications 1 à 5, **caractérisé en ce qu'**il contient des colorants montant directement sur la fibre, issus du groupe des nitrophénylènediamines, nitroaminophénols, anthraquinones, ou indophénols, de préférence en une quantité de 0,01 à 20% en poids par rapport au colorant total.

7. Agent selon l'une quelconque des revendications 1 à 6, **caractérisé en ce que** l'on ajoute des sels d'ammonium ou métalliques, choisis dans le groupe des formiates, carbonates, halogénures, sulfates, butyrates, valériates, caproates, acétates, lactates, glycolates, tartrates, citrates, gluconates, propionates, phosphates et phosphonates de métaux alcalins, comme le potassium, le sodium ou le lithium, de métaux alcalino-terreux, comme le magnésium, le calcium, le strontium ou le baryum, ou d'aluminium, de manganèse, de fer, de cobalt, de cuivre ou de zinc.

8. Agent selon l'une quelconque des revendications 1 à 7, **caractérisé en ce qu'**il contient des agents d'oxydation, en particulier H₂O₂, en une quantité de 0,1 à 6% en poids par rapport à la solution d'application.

9. Agent selon l'une quelconque des revendications 1 à 8, **caractérisé en ce qu'**il contient des tensioactifs anioniques, zwitterioniques ou non ioniques.

10. Utilisation d'une combinaison d'aldéhydes et de cétones hétéroaromatiques de formule I, 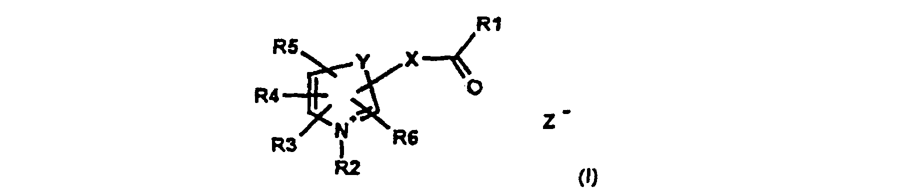 dans laquelle
R¹ représente un atome d'hydrogène, un groupe alkyle en C₁ à C₄, un groupe aryle, ou hétéroaryle,
R² représente un groupe alkyle en C₁ à C₄, un groupe aryle, aralkyle ou hétéroaryle,
R³, R⁴, R⁵ et R⁶ représentent un atome d'hydrogène ou d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyles en C₁ à C₄, ou un groupe acyle en C₁ à C₄, deux des résidus pouvant aussi former conjointement un noyau aromatique condensé ou bien un des résidus conjointement avec R¹ou avec R² pouvant former un cycle condensé à 5, 6, ou 7 maillons, qui peut porter également un noyau aromatique condensé,
X représente une liaison directe, un groupe vinylène, phénylène, ou vinylènephénylène, éventuellement substitué,
Y représente un atome de soufre, un atome d'oxygène,
le groupe CR⁷R⁸, dans lequel R⁷ et R⁸ représentent un groupe alkyle en C₁ à C₄, le groupe NR¹⁰, dans lequel R¹⁰ représente un groupe alkyle en C₁ à C₄, ou un groupe vinylène éventuellement substitué, et
Z⁻ représente un ion halogénure, alkylsulfate en C₁ à C₄, sulfate d'arène, alcane-sulfonate en C₁ à C₄, perfluoroalcane-sulfonate en C₁ à C₄, perhalogénate, sulfate, hydrogénosulfate, tétrafluoroborate, hexafluorophosphate, ou tétrachlorozincate,
et de nitroamines hétéroaromatiques de formule II, 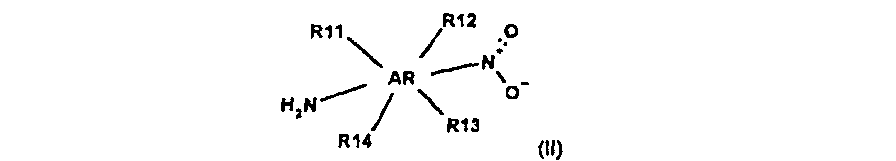 dans laquelle
AR représente un groupe pyridine, quinoléine, isoquinoléine, pyrimidine, indole, benzimidazole, benzothiazole, indazole, benzoxazole, quinoxaline, quinazoline, cinnoline, et
R¹¹, R¹², R¹³, et R¹⁴ représentent un atome d'hydrogène, un atome d'halogène, un groupe alkyle en C₁ à C₄, alcoxy en C₁ à C₄, aminoalkyle en C₁ à C₄, hydroxyalcoxy en C₁ à C₄, hydroxy, nitro, carboxy, (alcoxy en C₁ à C₄)-carbonyle, sulfo, sulfamoyle, arylazo ou amino, qui peut être substitué par des groupes alkyle en C₁ à C₄ ou hydroxyalkyle en C₁ à C₄,
et/ou un produit de réaction issu des composés de formule I et II, sous forme de composant colorant dans des agents colorants capillaires par oxydation.

11. Procédé pour colorer les fibres kératiniques, en particulier les cheveux humains, dans lequel un colorant, contenant au moins une combinaison d'aldéhydes et de cétones hétéroaromatiques de formule I, 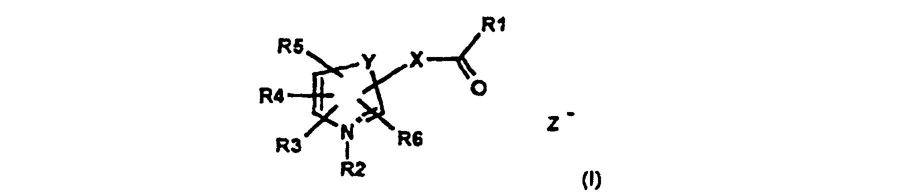 dans laquelle
R¹ représente un atome d'hydrogène, un groupe alkyle en C₁ à C₄, un groupe aryle, ou hétéroaryle,
R² représente un groupe alkyle en C₁ à C₄, un groupe aryle, aralkyle ou hétéroaryle,
R³, R⁴, R⁵ et R⁶ représentent un atome d'hydrogène ou d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyles en C₁ à C₄, ou un groupe acyle en C₁ à C₄, deux des résidus pouvant aussi former conjointement un noyau aromatique condensé ou bien un des résidus conjointement avec R¹ou avec R² pouvant former un cycle condensé à 5, 6, ou 7 maillons, qui peut porter également un noyau aromatique condensé,
X représente une liaison directe, un groupe vinylène, phénylène, ou vinylènephénylène, éventuellement substitué,
Y représente un atome de soufre, un atome d'oxygène,
le groupe CR⁷R⁸, dans lequel R⁷ et R⁸ représentent un groupe alkyle en C₁ à C₄, le groupe NR¹⁰, dans lequel R¹⁰ représente un groupe alkyle en C₁ à C₄, ou un groupe vinylène éventuellement substitué, et
Z⁻ représente un ion halogénure, alkylsulfate en C₁ à C₄, sulfate d'arène, alcane-sulfonate en C₁ à C₄, perfluoroalcane-sulfonate en C₁ à C₄, perhalogénate, sulfate, hydrogénosulfate, tétrafluoroborate, hexafiuorophosphate, ou tétrachlorozincate,
et de nitroamines hétéroaromatiques de formule II, 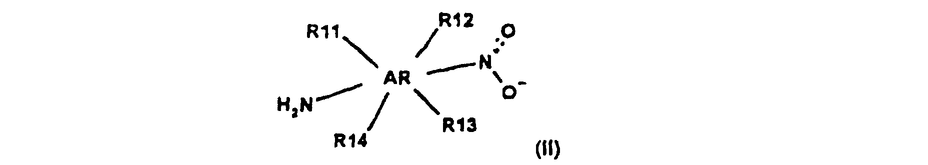 dans laquelle
AR représente un groupe pyridine, quinoléine, isoquinoléine, pyrimidine, indole, benzimidazole, benzothiazole, indazole, benzoxazole, quinoxaline, quinazoline, cinnoline, et
R¹¹, R¹², R¹³, et R¹⁴ représentent un atome d'hydrogène, un atome d'halogène, un groupe alkyle en C₁ à C₄, alcoxy en C₁ à C₄, aminoalkyle en C₁ à C₄, hydroxyalcoxy en C₁ à C₄, hydroxy, nitro, carboxy, (alcoxy en C₁ à C₄)-carbonyle, sulfo, sulfamoyle, arylazo ou amino, qui peut être substitué par des groupes alkyle en C₁ à C₄ ou hydroxyalkyle en C₁ à C₄,
et/ou un produit de réaction issu de ces composés, ainsi que des ingrédients cosmétiques courants, sont appliqués sur les fibres kératiniques, laissés quelque temps, habituellement pendant environ 30 min sur les fibres, puis éliminés par rinçage ou par lavage avec un shampoing.
